# EUROPEAN PATENT APPLICATION

(11) **EP 4 702 925 A1**
(43) Date of publication of application: **04.03.2026**
(21) Application number: 24382945.4
(22) Date of filing: 02.09.2024
(51) Int. Cl.: A61B 5/38, A61B 5/00

(54) **A COMPUTER IMPLEMENTED METHOD AND SYSTEM FOR PREDICTING A RISK THAT AN INFANT PRESENTS A NEURAL SPEECH ENCODING DISORDER**

(71) Applicant: Universitat de Barcelona, 08007 Barcelona (ES)
(72) Inventor: VALENZUELA RUIZ, Jose, 08028 Barcelona (ES); ESCERA MICO, Carles Enric, 08028 Barcelona (ES)
(74) Representative: ZBM Patents - Zea, Barlocci & Markvardsen

(57) **Abstract**

A system and computer implemented method of predicting a risk that an infant presents a neural speech encoding disorder is disclosed, the method comprising obtaining an electroencephalography signal of an infant in response to an auditory stimulus, obtaining a set of signal features from the obtained EEG signal, and predicting a risk that an infant presents a neural speech encoding disorder using a machine learning algorithm.

## Description

### FIELD OF THE INVENTION

The present disclosure relates to a computer implemented method and system for predicting a risk that an infant presents a neural speech encoding disorder using EEG signals.

### BACKGROUND

Neurodevelopment of infants has been studied in recent years by analysing EEG signals from infants. More specifically, predictions of the language neurodevelopment of an infant have been performed by studying frequency-following response (FFR) of auditory stimulus delivered to infants. The study of FFR has been previously used to predict short to long-term language acquisition delays and disabilities and disruptions. However, the existing methods are based on statistical calculations which are unprecise. Therefore, there is a need of a more precise and reliable method to predict disorders related to the neural encoding of language from a young age.

### SUMMARY

In a first aspect, a computer implemented method of predicting a risk that an infant presents a neural speech encoding disorder is provided. The method comprising the steps of:
- obtaining an electroencephalography, EEG, signal of an infant aged 0 to 6 months old in response to an auditory stimulus, wherein the auditory stimulus comprises a speech component representing at least two phonemes, wherein at least one phoneme is the 'a' phoneme;
- obtaining a set of signal features from the EEG signal, the set of signal features relating to the neural speech encoding performed by the brain of the infant;
- predicting a risk that an infant presents a neural speech encoding disorder using a machine learning algorithm, wherein the set of signal features is input to the machine learning algorithm;
wherein the machine learning algorithm is trained using a supervised training and a training data set, the training data set comprising:
- an input dataset comprising a set of signal features obtained from an EEG signal of each infant of a group of infants aged 0 to 6 months old; and
- an output dataset comprising a risk parameter for each infant of the group of infants, the risk parameter having at least two possible values, wherein a first value represents a high risk of a neural speech encoding disorder and a second value represents a low risk of a neural speech encoding disorder;
- wherein the value of the risk parameter is based on data corresponding to a Language scale of the Bayley test, the Language scale of the Bayley test being obtained from each one of the corresponding infants from which the set of signal features in the input dataset are obtained, and wherein the infants are aged 21 months old or older at the time of the obtaining of the Language scale of the Bayley test.

In the presently disclosed method, an EEG signal of a neonatal infant is obtained, the EEG signal corresponding to a neural response of the neonatal infant to the delivery of an auditory stimulus.

The auditory stimulus may be delivered to a neonatal infant (i.e., an infant of in between 0 and 6 months of age), the auditory stimulus being in the form of sound or sound wave. The auditory stimulus is a non-therapeutic auditory stimulus. The auditory stimulus may be represented, for example, by an auditory stimulus signal, the auditory stimulus signal being an electrical signal. The auditory stimulus may be delivered by, for example, sending the auditory stimulus signal to a sound-producing device. Such sound-producing device may be, for example, a pair of headphones, a single speaker or a single earphone, which produces a sound wave of the auditory stimulus from the auditory stimulus signal and delivers the sound wave to the infant.

The obtaining of an EEG signal in response to the delivery of an auditory stimulus may be referred to as the Frequency-Following Response (FFR) measurement. The obtained EEG signal comprises an encoding of the temporal and spectral characteristics of sounds processed by the cortico-subcortical auditory system of the brain of the infant. Such encoding of the auditory stimulus is found in the obtained EEG signal in the form of a reproduction of the auditory stimulus within the obtained EEG signal. Therefore, the EEG signal provides an indicator of the acoustic features of the speech components comprised in the auditory stimulus and can show the current functional state of the auditory system of the infant at the time of the obtaining of the EEG signal: the more similar the neural speech encoding represented by the obtained EEG signal is to the auditory stimulus, the more neurodevelopment the infant is considered to have, or in some examples, will be considered to have (i.e., less neural speech disorder the infant is considered to have). In some examples, neural speech disorders may comprise language acquisition delays.

This way, if a specific auditory stimulus is delivered to the infant (such as, for example, an auditory stimulus comprising speech components representing the Idal phoneme or the loal phoneme), the features of the auditory stimulus can be found reproduced (to some extent) in the obtained EEG signal. The EEG signal reflects the neural speech encoding of the auditory stimulus performed by the brain, the EEG signal acting as an "echo" of the auditory stimulus, and thus the auditory stimulus is reproduced to some extent on the obtained EEG signal. For example, the obtained EEG signal may have signal peaks at the frequencies corresponding to a fundamental frequency of the auditory stimulus.

After obtaining of the EEG signal, a set of signal features is obtained from the EEG signal. The signal features may be related to, for example, a parameter reflecting a frequency feature of the obtained EEG signal, or a temporal feature of the EEG signal. Then, a prediction is performed of the risk that the infant may present a neural speech encoding disorder, the prediction being performed from the obtained set of signal features, using a machine learning algorithm. The signal features obtained from the obtained EEG signal relate to the neural speech encoding performed by the brain of the infant.

The machine learning algorithm used to perform the prediction is pre-trained to output a risk parameter based on a set of signal features as explained herein below. The machine learning algorithm is pre-trained using a supervised training and a training data set, the training data set comprising:
- an input dataset comprising a set of signal features obtained from each infant of a group of infants aged 0 to 6 months old; and
- an output dataset comprising a risk parameter for each infant of the group of infants, the risk parameter having at least two possible values, wherein a first value represents a high risk of a neural speech encoding disorder and a second value represents a low risk of a neural speech encoding disorder;
- wherein the value of the risk parameter is based on data corresponding to a Language scale of the Bayley test, the Language scale of the Bayley test being obtained from each one of the corresponding infants from which the set of signal features in the input dataset are obtained, and wherein the infants are aged 21 months old or older at the time of the obtaining of the Language scale of the Bayley test.

Therefore, the input dataset comprises a group of sets of signal features. For example, for a group of 100 infants, the input dataset may comprise a group of 100 sets of signal features, each set of signal features corresponding to each one of the infants of the group of infants.

Furthermore, the output dataset comprises a group of risk parameters. For example, for a group of 100 infants, the output dataset may comprise a group of 100 risk parameters, each risk parameter corresponding to each one of the infants of the group of infants. A risk parameter may have at least two values corresponding to a high or a low risk of a neural speech encoding disorder. For example, the risk parameter may have a "0" value for a low risk and a "1" for a high risk. In another example, the risk parameter may be percentual, and values between 1 and 49 may correspond to a low risk, and values between 50 and 100 may correspond to a high risk. The risk parameter is obtained from data corresponding to a Language scale of the Bayley test, and the risk parameter may be computer-calculated based on said data or using a preset reference table of values relating the data corresponding to the Language scale with values of the risk parameter. The Bayley Test used to obtain the Language scale is performed on the infants of the group of infants from which the sets of signal features were obtained, at an age 21 months or older. The obtained EEG signal and the prediction of a neural speech encoding disorder within the first 21 months have a common neurophysiological basis, and hence they are mutually interdependent. In some examples, relevant linguistic milestones -for example, a small repertoire of several words- may have been reached at the age of 21 months. However, later in the life of the infant, the obtained EEG signal may reflect a distorting environmental influence (e.g., multilingual environment, socio-cultural and educational experience, i.e., milestones will no longer depend so much on neurobiology as on interaction with the environment). Failures in the brain mechanisms at birth and until 6 months, which may be found by using signal features from the obtained EEG signal as input data for the herein disclosed machine learning algorithm, anticipate neural speech encoding disorders at the age of 21 months and onwards. Said neural speech encoding disorders can be quantified by using a Language scale of the Bayley test as a training data set output for the same machine learning algorithm. The Bayley Scales of Infant and Toddler Development, known in short and from herein onwards as Bayley test, is an assessment test designed to measure motor, cognitive, language, social-emotional, and adaptive behaviour development in babies and young children.

The test involves performing an interaction between the infant and the examiner, which observes a series of non-therapeutic tasks completed by the infant.

As with other tests, the tasks range from basic responses to more complex responses from the infant. For example, a basic response may involve introducing an interesting object for the infant to track with their eyes. A more complex task might involve an infant finding hidden objects.

An infant may take about an hour to complete the entire Bayley test. After completing the series of tasks of the test, the examiner may produce a developmental quotient (DQ, which is different from an intelligence quotient or IQ).

The results of the Bayley test may be quantified in each one of a group of Bayley scales, the group of Bayley scales comprising the following scales:
- Cognitive Scale: Assesses problem-solving abilities, memory, and other cognitive skills. It measures an infant's ability to, for example, engage in pretend play, attend to objects, or look for an object that has fallen;
- Language Scale: Evaluates expressive and receptive language skills, including vocabulary and understanding of language. For example, it measures an infant's ability to understand and use spoken language to label objects or people, follow instructions, or recognize objects based on spoken description or labels;
- Motor Scale: Measures both fine and gross motor skills, including coordination, balance, and manipulation of objects;
- Social-Emotional Scale: Examines social interaction, emotional expression, and responsiveness to others. For example, it measures an infant's ability to engage with others socially, self-calm and how the infant takes part in age-appropriate play;
- Adaptive Behaviour Scale: Assesses the infant's ability to function in everyday activities and adapt to environmental demands. For example, it measures how the infant follows rules, cooperates, and generally adapts to new or demanding situations;

Each scale provides at least one score which indicates the infant's level of development compared to typical values for their age group. These scales help identify developmental delays or strengths in infants, guiding interventions and support for the infant's growth and learning.

Furthermore, the Language Scale comprises the following subscales:
- Receptive Communication subscale: It evaluates the infant's ability to understand language, including comprehension of spoken words and simple commands;
- Expressive Communication subscale: It evaluates the infant's ability to produce language, including vocabulary, use of gestures, and verbal expression;
- Gestures subscale: It specifically evaluates the infant's use of non-verbal communication, such as pointing or waving, which are early indicators of language development;
- Total Language Composite: It combines scores from the receptive and expressive communication subscales to provide an overall assessment of the infant's language development;

In the present method, any reference to the Language Scale of the Bayley Test may be understood as any one of the subscales of the Language Scale of the Bayley Test, which may also include the Total Language Composite subscale (which is a combination of the other subscales, and a "subscale" on its own).

Therefore, the machine learning algorithm is trained by inputting an input dataset into the algorithm which comprises a set of signal features obtained from EEG signals, the EEG signals being obtained from a group of infants aged 0-6 months in response to auditory stimulus individually delivered to each infant of the group of infants. Furthermore, the training involves, inputting each set of signal features in order to modify the algorithm to fit an output using an output dataset. The output dataset comprises a risk parameter corresponding to each set of signal features, the risk parameter being based on data corresponding to a Language scale of the Bayley test.

The risk parameter quantifies the risk that an infant presents a neural speech encoding disorder from the age 21 months or older. Furthermore, the Bayley test used to obtain said Language scale is performed on each one of the corresponding infants from which each set of signal features in the input dataset is obtained, when the infants are 21 months or older.

This way, the relationships between the set of signal features at a very early age of an infant (0 to 6 months old) and a Language scale of the Bayley test of the same children at age 21 months or older are established by the machine learning algorithm.

Furthermore, the machine learning algorithm can be used to predict a risk that an infant presents a neural speech encoding disorder by inferring the risk parameter with a new set of signal features, the set of features obtained from the EEG signal of an infant aged 0 to 6 months as a response to the delivery of an auditory stimulus.

This is possible because the obtained EEG signal represents how a neonatal infant encodes speech sounds in its brain. The speech encoding performed by the brain of the infant consequently influences how the infant encodes phonemes, which in turn will influence (in time) how the infant subsequently processes language, and the risk of a neural speech encoding disorder occurring.

By using the presently disclosed method, early detections of future neural speech encoding disorders in neonatal infants may be achieved, thus helping to palliate derail in the infant's future neurodevelopment. Machine learning (ML) algorithms may be applied in this field to reduce time analysis and to identify correlations between signal features from an EEG signal and one or more subscales within a Language scale of the Bayley test.

Human language acquisition relies on a predefined development of the auditory cortex of the human brain, which, in a fetus, is already functional to process sounds at the beginning of the third trimester of pregnancy. Around the 27th gestational week, first traces of myelin can be observed in both the cochlear nerve and the brainstem auditory pathway. At birth, the cochlea -the peripheral organ of hearing- has reached its adult size and is fully functional, but the auditory cortex is not completely mature yet, thus developing during the first year of life of the infant.

The formation of myelin sheaths around the axons of neurons in the brain of an infant - myelination - starts during fetal development, the sheaths not being fully formed until the age of one year old. Myelin sheaths are particularly important on long axonal fibres, the fibres having long projections of neurons within the nervous system of the human brain. Long axons are found, for example, connecting relatively distant parts of the brain, the distant parts benefitting from myelination due to the need for rapid and efficient signal transmission over long distances. Therefore, myelination in long axonal fibers is important for neural circuits involved in speech production and language comprehension, which cover relatively distant parts of the brain. More specifically, myelin enhances the ability of an electrical impulse, travelling along the axon, to jump from one node of Ranvier to the next, significantly speeding up transmission between neurons. Therefore, myelination of long axonal fibres within the brain is important in the development of speech production and language comprehension in neonatal infants by facilitating efficient neural communication within the brain.

The areas of the brain involved in Language and Speech are the following:
- The primary and association auditory cortices of the temporal lobe, primarily involved in processing the speech sounds and another features of speech, such as intonation and prosody.
- Broca's Area: Located in the left frontal lobe of the brain, this area is primarily involved in speech production and language comprehension.
- Wernicke's Area: Found in the temporal lobe of the brain, it is crucial for language comprehension.
- Arcuate Fasciculus: A bundle of axonal fibres connecting Broca's and Wernicke's areas, essential for integrating speech production and language comprehension.

Demyelination in the above-listed areas of the brain can disrupt normal myelin function, leading to impaired nerve signal transmission, among other consequences, which may result in a variety of neurological symptoms. The symptoms are quantifiable by one or more signal features of an EEG signal of the affected brain, in response to an auditory stimulus, since the EEG signal reflects the neural speech encoding performed by the neurons found within the above-listed areas of the brain. In the case of neonatal infants between 0 and 6 months, the obtained EEG signal in response to an auditory stimulus has a low signal-to-noise ratio (SNR), high inter-subject variability, and is a non-stationary signal. Therefore, statistical measurements of said EEG signal, in the case of neonatal infants between 0 and 6 months, are imprecise and not suitable for predicting a risk that an infant presents a neural speech encoding disorder.

A critical factor in neural speech encoding in neonatal infants is the resolution of minute temporal differences of speech (in the order of a millisecond or less), which require precise and accurate neural communication, enabled by myelin sheaths formation. Minute temporal differences in speech may refer to the ability to detect and process relatively small time-intervals between sounds or phonetic elements in spoken language. This capability is crucial for distinguishing between different speech sounds, understanding the rhythm and intonation of speech, and accurately perceiving spoken words and sentences. High temporal resolution allows the auditory system to differentiate closely spaced sounds, such as the difference between "pa" and "ba," which helps in the accurate comprehension of speech.

The obtained EEG signal in response to an auditory stimulus reflects one or more of at least the following four independent brain mechanisms related to speech encoding:
- Processing speed;
- Pitch encoding;
- Fine-grained spectro-temporal processing; and
- Neural noise.

Processing speed, as quantified by a neural lag signal feature of the obtained EEG signal, reflects the time conduction necessary for the neural signals in the different auditory relays to travel from one to another, and depends on long axonal fibres myelinization within the auditory nerve and in the ascending auditory pathway. As axonal myelin matures with age, neural lag shortens, and hence, abnormal neural lags at birth, due to defective myelinization in utero, may anticipate later neural speech encoding disorders, as reflected in the Language Bayley's scale.

Pitch encoding, as quantified by a pitch tracking signal feature of the obtained EEG signal. The pitch tracking signal comprises the measurement of the spectral amplitude of the obtained EEG signal at the fundamental frequency of the auditory stimulus, and its corresponding SNR at said fundamental frequency. This signal feature reflects the neural phase-locking to the periodicity (envelope) of the auditory stimulus happening in the subcortical auditory pathway, and partially in the auditory cortex up to 150-200 Hz, corresponding to voice pitch (the fundamental frequency of the human voice). Neural phase-locking has been shown to mature during the first years of life as both the subcortical and cortical auditory systems mature in terms of myelinization development. The myelinization of those auditory systems can be impaired due to genetic, nutritional, traumatic, and environmental factors, even during fetal development, and hence lead to deficient neural phase-locking from birth to 6 months. A lower myelinization can be quantified by, for example, a low SNR, which reflects a possible future neural speech encoding disorder.

Fine-grained spectro-temporal processing, as quantified by a feature signal comprising the spectral amplitude signal of the obtained EEG signal at the frequency corresponding to the first formant of the auditory stimulus (F1 of the vowel or vowels of the auditory stimulus) and the corresponding SNR at said frequency. This feature signal reflects the ability of neural circuits between neurons to track the minute temporal variations between two consecutive cycles in the auditory stimulus implicated in phoneme discrimination (i.e., ranging from 0.3 to 1 ms). This tracking requires a precise neural communication within the brain allowing phase encoding, which relies on a proper myelination of specific parts of the brain (for example, at the axons projecting to short distances within a cortical column). Said myelination depends on a distinct type of myelin-production cells in the central nervous systems, and alterations in fine-grained spectro-temporal processing at age 0 to 6 months reflect a possible future neural speech encoding disorder.

Neural noise, as quantified by a root mean square (RMS) signal feature of the obtained EEG signal, and its SNR. Both signal features provide a measure of the background noise in the obtained EEG signal and thus a measure of brain complexity. Neural noise increases with age, in parallel with the increased connectivity of the cortical mantle. Defective (or excessive) neural noise at birth until 6 months old may anticipate a future neural speech encoding disorder.

Thus, as exemplified by the above brain mechanisms and the signal features which reflect them, an abnormally low or high myelination of the areas of the brain involved in Language and speech may affect the above-listed signal features of an obtained EEG signal in response to the delivery of an auditory stimulus to a neonatal infant of between 0 and 6 months.

A higher myelination increases the speed of electrical impulse conduction along axons. This rapid transmission allows for better synchronization of neural activity across different regions of the brain of the neonatal between 0 and 6 months, which in turn leads to more coherent and higher frequency obtained EEG signals, especially in the alpha (8-12 Hz) and beta (13-30 Hz) bands. Neural activity in these bands is typically associated with myelinated fibres in a mature brain and reflect efficient neural processing and communication.

On the other hand, a below-average myelination of the areas of the brain involved in Language and Speech decreases the speed of neural conduction along axons and makes it less synchronized. This can result in activity in the Theta (4-7 Hz) and Delta (0.5-3 Hz) bands, and a reduced coherence in the obtained EEG signal. These bands are more prominent in neonatal infants and young children, whose brains are still undergoing myelination.

Therefore, during early development, i.e., in neonatal infants from 0 to 6 months, obtained EEG signals show predominant signal power in the Theta and Delta bands due to ongoing myelination and, as the brain matures and myelination progresses, there is a gradual increase in signal power in the Alpha and Beta bands.

Therefore, by using a machine learning algorithm trained with inputs comprising signal features, either in the temporal or spectral domain, different characteristics of the neurophysiology underlying speech encoding can be retrieved. More specifically, the obtained EEG signal from a neonatal infant comprises a plurality of signal features which reflect at least four independent brain mechanisms: timing, pitch encoding, fine-grained spectro temporal processing, and neural noise.

In an example, the method of predicting a risk that an infant presents a neural speech encoding disorder further comprises:
- obtaining at least two portions of the obtained EEG signal, the at least two portions having an alternate polarity in time between them;
- obtaining an averaged EEG signal by averaging the obtained at least two portions;
and wherein the set of signal features is obtained from the averaged EEG signal.

In this example, the auditory stimulus is represented by an auditory stimulus signal comprising at least two portions in time, wherein the portions have an alternate polarity in time between them.

For example, the auditory stimulus signal may comprise a first portion comprising a speech component representing the phoneme |oa| with a positive polarity, and a second portion comprising a speech component representing the phoneme loal with a negative polarity. When the auditory stimulus signal is input in a sound reproducing device (such as, for example, an earphone or a speaker) equipped with a diaphragm, the auditory stimulus signal causes that the device's diaphragm, emitting the auditory stimulus, to move between an outward position -in an outward movement- and an inward position -in an inward movement-. The inward and outward movements generate an acoustic condensation caused by the outward movement of the device's diaphragm and rarefaction caused by the inward movement of the device's diaphragm of the sound wave corresponding to the auditory stimulus signal.

The averaging of the at least two portions of the obtained EEG signal allows preprocessing the EEG signal such that a precise calculation of a specific signal feature is achieved.

In an example, a plurality of portions of the obtained EEG signal may be obtained, a first set of the plurality of portions having an alternate polarity with respect of a second set of the plurality of portions, and the average EEG signal being obtained by averaging the plurality of portions. Thus, for example, an auditory stimulus signal representing an auditory stimulus may have 100 speech components representing the phoneme |oa|, one after the other in time. For example, each of the 100 speech components may have an alternate polarity, i.e., the first may have a positive polarity, the second may have a negative polarity, the third may have a positive polarity, and so on. In this example, the average may be performed over all the 100 speech components of the auditory stimulus signal, which are found as a response to the stimulus, within the obtained EEG signal. In another example, with the same auditory stimulus signal comprising 100 speech components, the first 50 speech components may have a positive polarity, and the following 50 speech components may have a negative polarity.

The averaging may be performed using different averaging methods. In an example, the averaging may be performed by subtracting each obtained portion of the first set from another obtained portion of the second set, and dividing the result of each subtraction by two. For example, in the example where the auditory stimulus signal comprises 100 speech components with an alternate polarity in time between each other (first positive, second negative, third positive, and so on), the averaging may be performed by selecting adjacent pairs of portions (for example, first and second, third and fourth, and so on) and subtracting, for each pair, one portion of the pair from the other one, dividing the result by two and adding all the results of each subtraction and division, in order to obtain an averaged EEG signal. In this case, by performing the described averaging comprising a subtraction with pairs of portions having alternate polarities between the portions of the pair, the power of the obtained EEG signal at the fundamental frequency of the auditory stimulus is decreased, and thus the power at the frequencies of the formants of the auditory stimulus is enhanced on the resulting averaged EEG signal.

Alternatively, in an example, the averaging may be performed by adding each obtained portion of the first set from another obtained portion of the second set, and dividing the result of each subtraction by two. For example, in the example where the auditory stimulus signal comprises 100 speech components with an alternate polarity in time between each other (first positive, second negative, third positive, and so on), the averaging may be performed by selecting adjacent pairs of portions (for example, first and second, third and fourth, and so on) and adding, for each pair, one portion of the pair to the other one, dividing the result by two and adding all the results of each addition and division, in order to obtain an averaged EEG signal. In this case, by performing the described averaging comprising an addition with pairs of portions having alternate polarities between the portions of the pair, the power of the obtained EEG signal at the frequency of the formants of the auditory stimulus is decreased, and thus the power at the fundamental frequency of the auditory stimulus is enhanced on the resulting averaged EEG signal.

Selecting either averaging (e.g., by subtracting or by adding) allows obtaining signal features relating either to the formants' frequencies (in the case of "by subtracting") or to the fundamental frequencies (in the case of "by adding"). For example, the signal-to-noise ratio of the resulting averaged EEG signal is increased from the originally obtained EEG signal at a desired frequency (either the formants' frequencies or the fundamental frequencies), because of the cancelling or minimizing of other signals within the obtained EEG signal.

The previously described averaging calculations may also be performed, in an example, over a plurality of pairs of portions obtained from the obtained EEG signal, wherein the portions are not adjacent in time. As long as each pair of portions used to either perform a subtraction or an addition have an alternate polarity between the portions of the pair, the resulting technical effect of decreasing either the power of the EEG signal at the fundamental frequency of the auditory stimulus or at the frequencies of the formants of the auditory stimulus may also occur.

The signal features relating to the neural speech encoding performed by the brain of the infant may comprise, in some examples, a temporal signal feature. The temporal signal features may comprise, for example, at least one of:
- Root Mean Square (RMS) of the obtained EEG signal: the RMS of the obtained EEG signal is calculated as the square root of the mean of the squared values within the obtained EEG signal;
- Root Mean Square (RMS) of the baseline is the RMS of the baseline. In some examples, the baseline is a temporal window of EEG recording taken just before the presentation of each auditory stimulus (its value is therefore negative to the time 0 of the stimulus, i.e. -40ms - 0ms). It may be used as a reference to observe the disturbance caused by the auditory stimulus in the EEG (for example, reflected by the phase-locking of the auditory stimulus). It can also be used to calculate other parameters, such as the SNR of the RMS in response to the auditory stimulus. Thus, for example, for an auditory stimulus of 100ms, the RMS of the baseline may be obtained between -40ms and 0ms and of the auditory stimulus between 0ms and 100ms, and the SNR may be calculated by dividing one by the other;
- Root Mean Square (RMS) ratio: It is calculated as the ratio between the RMS of the obtained EEG signal and the RMS of the baseline of the obtained EEG signal;
- Maximum stimulus-response cross-correlation: it is the maximum of the cross-correlation function between the auditory stimulus signal used to generate the auditory stimulus and the obtained EEG signal within a temporal window. The window may be of between, for example, 3 and 13ms;
- Neural lag: it is defined as the moment in time in which the stimulus-response cross-correlation reaches a maximum. It is constrained to be found, for example, between 3 and 13ms;
- Pitch strength: it is calculated as the maximum value of the normalized autocorrelation of the obtained EEG signal.

The signal features relating to the neural speech encoding performed by the brain of the infant may comprise, in some examples, a frequency signal feature. The frequency signal features may comprise, for example, at least one of:
- Spectral amplitude: it may be calculated as the amplitude of the frequency spectrum of the obtained EEG signal or the averaged EEG signal, the spectral amplitude being measured at a predetermined frequency of the auditory stimulus. For example, it may be calculated as the averaged value of the amplitude of the FFT of the obtained or averaged EEG signal within a selected frequency range, retrieved from the frequency spectrum of the obtained or averaged EEG signal. In some examples, the selected frequency range may be centered at the fundamental frequency of the auditory stimulus, or around the frequency of a formant of the auditory stimulus signal (for example, F₁);
- Signal-to-noise ratio (SNR): it may be calculated as the ratio between the spectral amplitude of the obtained EEG signal and the noise around it, at the fundamental frequency of the auditory stimulus.

In an example, the machine learning algorithm may be a Linear Models type algorithm. A linear model is a type of machine learning algorithm that assumes a linear relationship between the input features of the algorithm and the target variable. The defining characteristic of a linear model is that the model parameters are linearly combined with the input features to make predictions. The parameters used to train the model are linearly correlated with the level of language neurodevelopment, and therefore a linear model can be fit to the linear correlations. For example, if a set of signal features comprises the Signal-to-noise ratio (SNR) and Neural lag features, it is known that a higher value of SNR and a low Neural lag reflects a better neural encoding of the auditory stimulus performed by the brain of an infant, which enhances the process of language acquisition of the infant and decreases the risk of a neural speech encoding disorder. Therefore, a linear model may be more useful to identify the relationship between features and a specific value of risk parameter of the above-mentioned example.

In an example, the value of the risk parameter may be based on data corresponding to the Expressive Communication subscale of the Language scale of the Bayley test.

In an example, the Linear Model type algorithm may be one of:
- Logistic Regression (LR): A linear model used for binary classification problems.
- Naive Bayes (NB): It's based on Bayes' theorem and assumes independence between features.

Logistic Regression algorithms predict the probability that a given input belongs to a particular class. This makes it useful when probabilistic interpretation of results is required. Furthermore, they model the relationship between the independent variables and the log-odds of the dependent variable via a linear equation. This implies a linear decision boundary.

Naive Bayes algorithms assign the class label with the highest probability. This decision is made based on a linear combination of the input features and their associated probabilities. Although the algorithm assumes feature independence (which is a naive assumption), the classification decision is ultimately based on linear combinations of the logarithms of these probabilities.

While Naive Bayes doesn't involve explicit estimation of parameters like in linear regression, its decision boundary is linear when considering the logarithms of probabilities. Therefore, it's categorized alongside linear models due to its mathematical characteristics and simplicity.

In an example, the machine learning algorithm is an Instance-based type algorithm. This type of methods, also known as instance-based learning or lazy learning, are a type of machine learning algorithm that makes predictions based on similarities between new data instances and existing training instances. Unlike other algorithms that learn a model during the training phase, instance-based methods store the training data and make predictions only when a new instance is encountered.

In some examples, the Instance-based type algorithm may be a K-Nearest Neighbors (KNN) algorithm. A KNN algorithm is a non-parametric method that classifies data points based on the majority class among their k-nearest neighbors. KNN is robust to noisy data (such as any biological signal, as for example an EEG signal) when using a Grid Search engine to adjust hyperparameters and the K value.

In an example, the machine learning algorithm is a Kernel type algorithm. Kernel type algorithms are a class of machine learning algorithms that operate implicitly in a high-dimensional feature space using a kernel function. These methods are particularly useful for tasks such as classification, regression, and clustering, where linear decision boundaries or simple models may not be sufficient to capture the underlying patterns in the data.

In an example, the Kernel type algorithm is a Support Vector Machine (SVM). SVM algorithms construct a hyperplane or set of hyperplanes in a high-dimensional space that can be used for classification, regression, or other tasks. SVMs are effective in high-dimensional spaces, such as datasets comprising EEG signals, which comprise a lot of different temporal and frequency characteristics. Furthermore, SVMs are versatile due to its kernel trick and perform well with medium or small sized datasets.

In an example, the value of the risk parameter may be based on data corresponding to the Receptive Communication subscale of the Language scale of the Bayley test, and the machine learning algorithm may be a Tree-Based type algorithm. This type of algorithms constructs decision trees to make predictions based on input features. Tree-based type algorithms are versatile and widely used in various machine learning applications due to their ability to handle complex datasets, provide interpretable models, and achieve high predictive accuracy with proper tuning and regularization. However, they may be prone to overfitting, especially when the trees are deep or when the dataset is noisy or contains irrelevant features. Regularization techniques, ensemble methods, and hyperparameter tuning can help mitigate these issues and improve the performance of tree-based models.

In an example, the Tree-Based type algorithm is one of a:
- Decision Tree Classifier (DTC): it constructs a decision tree by recursively splitting the data based on feature values to create nodes that represent class labels. DTCs partition the feature space into regions, making them less sensitive to noise and outliers and able to capture non-linear relationships. This flexibility allows DTCs to model complex interactions between the signal features of the obtained or averaged EEG signals, which are related to the risk of a a neural speech encoding disorder.
- Random Forest (RF): it is an ensemble learning method that constructs multiple decision trees during training and outputs the mode of the classes (classification) or the mean prediction (regression) of the individual trees. RFs are effective in handling high-dimensional data, such as EEG signals with multiple temporal and frequency features. They automatically perform feature selection by considering subsets of features at each split, which can help mitigate the curse of dimensionality.
- Extreme Tree Classifier (ETC): it is similar to an RF algorithm, but uses extremely randomized trees, which means splitting points are selected at random. Similar to Random Forests, Extreme Tree Classifier aggregates predictions from multiple decision trees. However, Extreme Trees introduce additional randomness in the tree-building process by selecting random thresholds for each feature at each split point. This extra randomness helps reduce variance and can lead to better generalization performance.
- XGBoost (XGB): it is an implementation of gradient boosting that builds multiple trees in sequence, each one correcting errors of its predecessors. It incorporates regularization techniques to prevent overfitting. It can also capture complex relationships between signal features from the obtained EEG signal and the target variable (in this case, a Language scale of the Bayleys value). By iteratively training weak learners to correct the errors of previous models, XGBoost can model intricate patterns in the input data, which are essential for predicting neurodevelopment outcomes from EEG signals.

In another aspect, a computer program product is disclosed. The computer program product may comprise program instructions for causing a computing system to perform a method of predicting a risk that an infant presents a neural speech encoding disorder, according to some examples disclosed herein.

The computer program product may be embodied on a storage medium (for example, a CD-ROM, a DVD, a USB drive, on a computer memory or on a read-only memory) or carried on a carrier signal (for example, on an electrical or optical carrier signal).

The computer program may be in the form of source code, object code, a code intermediate source and object code such as in partially compiled form, or in any other form suitable for use in the implementation of the processes. The carrier may be any entity or device capable of carrying the computer program.

For example, the carrier may comprise a storage medium, such as a ROM, for example a CD ROM or a semiconductor ROM, or a magnetic recording medium, for example a hard disk. Further, the carrier may be a transmissible carrier such as an electrical or optical signal, which may be conveyed via electrical or optical cable or by radio or other means.

When the computer program is embodied in a signal that may be conveyed directly by a cable or other device or means, the carrier may be constituted by such cable or other device or means.

Alternatively, the carrier may be an integrated circuit in which the computer program is embedded, the integrated circuit being adapted for performing, or for use in the performance of, the relevant methods.

### BRIEF DESCRIPTION OF THE DRAWINGS

Non-limiting examples of the present disclosure will be described in the following, with reference to the appended drawings, in which:
Figure 1 depicts an example of a system for predicting a risk that an infant presents a neural speech encoding disorder, according to the present disclosure.
Figure 2 depicts an example of the method of predicting a risk that an infant presents a neural speech encoding disorder, according to the present disclosure.
Figure 3 shows an example of a speech component representing the |oa| phoneme in time, according to the present disclosure.
Figure 4 depicts a detail of the database 13 of the example of figure 1, according to the present disclosure.
Figure 5 depicts an example of the training process of a machine learning algorithm, according to the present disclosure.
Figure 6 depicts another example of a system for predicting a risk that an infant presents a neural speech encoding disorder, according to the present disclosure.
Figure 7 depicts another example of the method of predicting a risk that an infant presents a neural speech encoding disorder, according to the present disclosure.

### DETAILED DESCRIPTION OF EXAMPLES

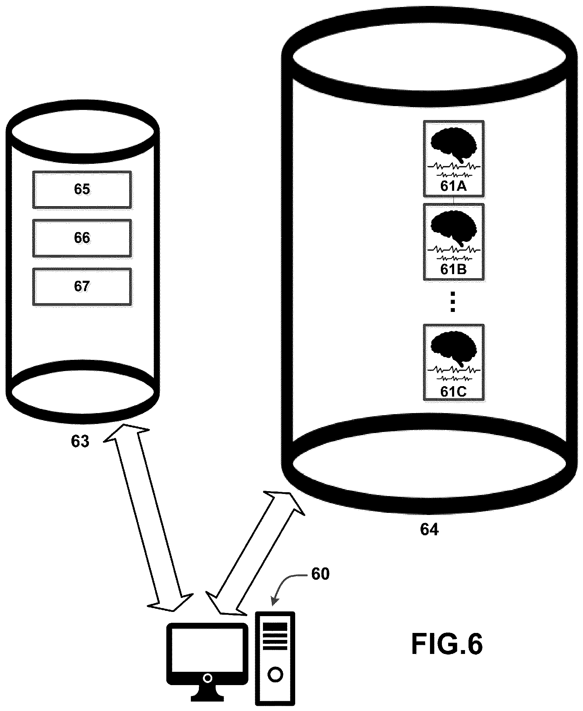

Figure 6 depicts an example of a system for predicting a risk that an infant presents a neural speech encoding disorder, according to the present disclosure. The system comprises a computing system 60 in communication with a first database 63 and a second database 64, the first database 63 comprising a data block 65 comprising a machine learning algorithm (in this example, a Logistic Regression model), a data block 66 comprising a group of sets of signal features, and a data block 67 comprising metadata related to the each of the sets of the group of sets of signal features of data block 66. Furthermore, the second database comprises a group of EEG signal files 61A, 61B and 61C, the EEG signal files corresponding to EEG signals previously obtained in response to the delivery of an auditory stimulus to an infant, each EEG signal file 61A, 61B and 61C corresponding to different infants.

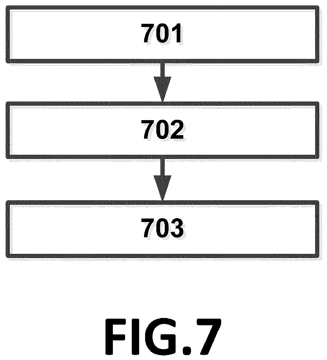

Figure 7 depicts an example of the method of predicting a risk that an infant presents a neural speech encoding disorder, according to the present disclosure, as performed by the system of figure 6.

In step 701, an electroencephalography, EEG, signal of an infant aged 0 to 6 months old is obtained by the computing system 60, retrieving it from database 64, in the form of an EEG signal file 61A. The EEG signal embodied in the EEG signal file 61A has been previously obtained in response to the delivery of an auditory stimulus to the infant, wherein the auditory stimulus comprises a speech component representing at least two phonemes, wherein at least one phoneme is the 'a' phoneme.

In step 702, the computing system 60 calculates a set of signal features from the obtained EEG signal file 61A. The calculated set of signal features relate to the neural speech encoding performed by the brain of the infant.

In step 703, a prediction of the risk that an infant presents a neural speech encoding disorder is performed by the computing system 60. The prediction is performed by the computing system 60 using the Logistic Regression model 65 found within database 63, using the calculated set of signal features as an input to the machine learning algorithm 65.

In this example, the Logistic Regression model 65 has been previously trained using a supervised training and a training data set, the training data set comprising:
- an input dataset comprising the group of sets of signal features found in data block 66, each set of signal features being obtained from an EEG signal of each infant of a group of infants aged 0 to 6 months old;
- an output dataset comprising a risk parameter for each infant of the group of infants, the risk parameter having at least two possible values, wherein a first value represents a high risk of a neural speech encoding disorder and a second value represents a low risk of a neural speech encoding disorder. Furthermore, the value of the risk parameter is based on data corresponding to a Language scale of the Bayley test, the Language scale of the Bayley test being obtained from each one of the corresponding infants from which each set of signal features found in data block 66, in the input dataset of the Logistic Regression model 65, are obtained, and wherein the infants were aged 21 months old or older at the time of the obtaining of the Language scale of the Bayley test.

Therefore, in step 703, the prediction performed by the computing system 60 is embodied in the output of the Logistic Regression model 65, in the form of a risk parameter, as used in the training of the model 65. The risk parameter corresponds to the input used to perform the prediction, i.e., the set of signal features calculated in step 702.

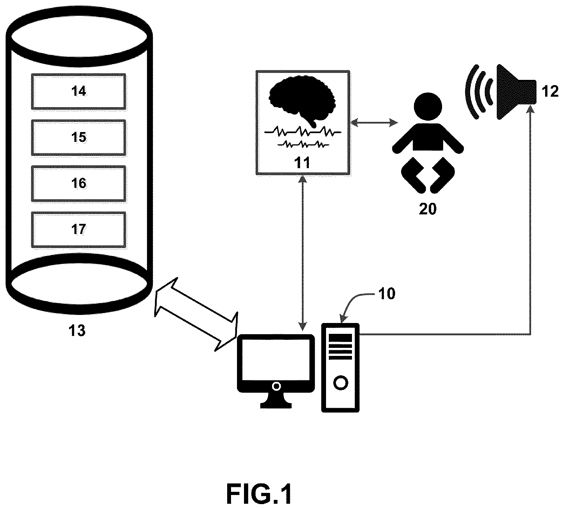

Figure 1 depicts an example of a system for predicting a risk that an infant presents a neural speech encoding disorder, according to the present disclosure. The system comprises a computing system 10 in communication with an EEG measurement device 11 and an earphone 12. The earphone 12 is placed nearby a neonatal infant's 20 ear, and the EEG measurement device 11 is placed in such a way that it obtains the neural activity of the neonatal infant 20. The computing system 10 is also connected to a database 13, the database 13 comprising a data block 14 comprising a machine learning algorithm, a data block 15 comprising a group of sets of signal features and a data block 16 comprising metadata related to the each of the sets of the group of sets of signal features of data block 15.

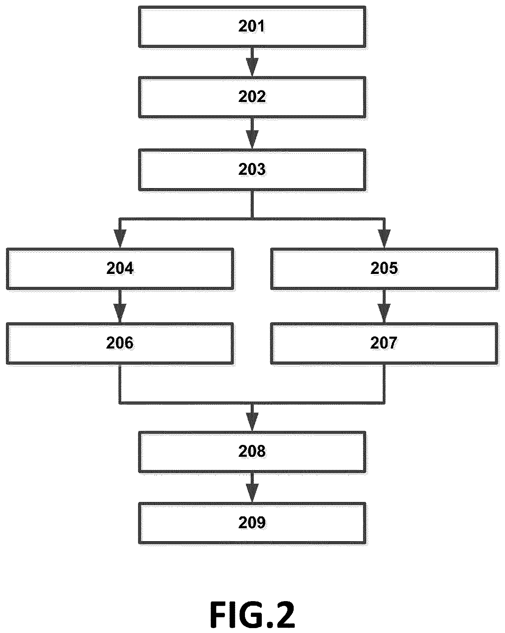

Figure 2 depicts an example of the method of predicting a risk that an infant presents a neural speech encoding disorder, according to the present disclosure, as performed by the system of figure 1.

In step 201 of the method, an auditory stimulus is generated by the computing system 10 in the form of an electrical auditory stimulus signal, the signal being sent to the earphone 12, which is worn by the neonatal infant 20 which, in this example, is 3 months old. The earphone 12 transforms the generated auditory stimulus signal into a sound wave, which is delivered by the earphone 12 to the infant 20.

In this example, the auditory stimulus delivered to the infant 20 is based on an auditory stimulus signal generated and stored within the computing system 10, which comprises 50 repetitions of the speech component representing the |oa| phoneme.

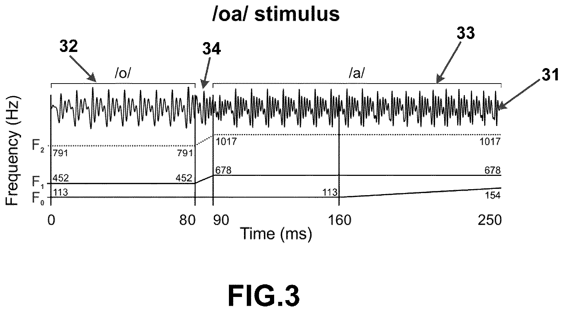

Figure 3 shows one of the 50 repetitions in time of the speech component 31 representing the |oa| phoneme in time, found within the auditory stimulus signal sent to the earphone 12, to deliver the auditory stimulus to the infant 20 in the example of figure 1 and 2. In figure 3, the speech component 31 is depicted in time and amplitude, and a plurality of frequencies is depicted with a value which remains or changes in time, in parallel to the speech component 31. More precisely, as seen in figure 3, the speech component 31 has a fundamental frequency F0 and a plurality of formants (in this figure only formants F1 and F2 are depicted). F0, F1 and F2 are represented in their value in Hz, and how they change in time through the |oa| phoneme.

The fundamental frequency of a signal (such as, for example, an auditory stimulus signal representing an auditory stimulus, the speech component 31 within an auditory stimulus signal, an EEG signal or an auditory stimulus in sound wave form) is defined as the lowest frequency of a periodic waveform within the signal. Furthermore, the fundamental frequency of an auditory stimulus is the lowest resonant frequency of the vibration of, for example, an instrument, device, or vocal cords producing the auditory stimulus (in the example of figure 1, the earphone 12 transduces the auditory stimulus signal sent from the computing system 10 into the auditory stimulus delivered to the infant 20, in the form of a sound wave). As a result of this, the fundamental frequency may be perceived as the loudest frequency of the auditory stimulus, while other frequencies are not heard separately but blended together by the ear into a single sound.

Furthermore, an auditory stimulus may also comprise formants (such as F0, F1 and F2 of the speech component 31 of the example of figure 3), which are frequency peaks in the spectrum of the auditory stimulus which have a high degree of energy compared to the rest of the auditory stimulus. Formants are especially prominent in phonemes corresponding to vowels, and each formant corresponds to a resonance in the human vocal tract that produces the phoneme, when the auditory stimulus signal is produced by a human being.

In speech production, the vocal tract acts as a filter, modifying the sound produced by the vocal cords to create different speech sounds. Each vowel sound is characterized by a specific pattern of formants, which are typically labelled as F1, F2, F3, and so on, in order of increasing frequency. More specifically, these three formants correspond to:
- F₁: It generally corresponds to the openness of the vocal tract. Higher F₁ frequencies are associated with more open vowel sounds;
- F₂: It corresponds to the front-to-back position of the tongue in the mouth. Higher F₂ frequencies are associated with front vowel sounds, while lower frequencies are associated with back vowel sounds;
- F₃: Less consistently associated with specific articulatory features, but may provide additional information about vowel quality, especially in certain languages or speech contexts.

An example of formants may be, for the /o/ phoneme corresponding to the vowel "o", F1 = 452 Hz and F2 = 791 Hz.

The speech component 31 used to deliver the auditory stimulus to the infant 20 of the example of figure 1 comprises a first portion 32 corresponding to the phoneme |o|, and a second portion 33 corresponding to the phoneme |a|, which together form the speech component 31 corresponding to the |oa| phoneme. Furthermore, as seen in figure 3, a transition period 34 is also found in the speech component 31, between the |o| and the |a| phonemes. In this example, the |o| phoneme is delivered from time 0ms to time 80ms, the transition period 34 is delivered in the subsequent 10ms, and the |a| phoneme is delivered from time 90ms to time 250ms. Fundamental frequency F0 and formant frequencies F1 and F2 evolve while the phonemes evolve in time.

As it can be seen in figure 3, the |oa| phoneme comprises a fundamental frequency F0 of 113 Hz, wherein its value stays stable during the |o| phoneme and the |a| phoneme, but wherein at time 160ms (approximately half through the |a| phoneme) F0 starts to rise until reaching 154Hz by the end of the delivery of the |oa| phoneme at time 250ms.

Also, it can be seen in figure 3, the |oa| phoneme further comprises formants F1 and F2. Such formants correspond to a single phoneme, and thus they vary within each single phoneme. More precisely, the frequency of the first formant F1 of the |o| phoneme stays at 452 Hz during the length of the |o| phoneme, and, in the |o| phoneme, the value of F1 coincides with the fourth harmonic frequency of the |o| phoneme. The frequency of the first formant F1 of the lal phoneme stays at 678 Hz during the length of the |a| phoneme, and, in this case, the value of F1 coincides with the sixth harmonic frequency of the |a| phoneme. Furthermore, the frequency of the second formant F2 of the |o| phoneme stays at 791 Hz during the length of the |o| phoneme, and the frequency of the second formant F2 of the lal phoneme stays at 1017 Hz during the length of the |a| phoneme.

The amplitude and length of the auditory stimulus signal, and the values of each of the above-listed frequencies of the auditory stimulus signal are analyzed and stored when the auditory stimulus signal is generated and stored within the computing system 10 in the example of figure 1.

The auditory stimulus signal is thus generated repeating the speech component 31 in time for 50 times. Before sending the 50 repetitions of the speech component 31, the 50 repetitions are multiplied by a string of alternating values of +1 and -1. Therefore, the auditory stimulus signal is transformed into an electrical signal which has an alternating polarity in time, which alternates between a positive and a negative polarity when transformed into a sound wave by the earphone 12. The frequency of the alternation of the polarity is equal to the frequency of changes between each repetition of the speech component 31, i.e., each speech component 31 of the 50 speech components 31 forming the auditory stimulus signal alternates between a positive and a negative value along the 50 repetitions (1st repetition positive, 2nd repetition negative, 3rd repetition positive, etc...).

Once the generated auditory stimulus signal is transformed into a sound wave by the earphone 12, and thus delivered to the infant 20, in step 202, an electroencephalography, EEG, signal is obtained from the infant 20 using the EEG device 11, in response to the auditory stimulus previously delivered to the infant 20 by the computing system 10.

In step 203, the calculation of a set of signal features is started by the computing system 10, the calculation being performed from the obtained EEG signal received from the EEG device 11. The calculation of the signal features is also based on the data corresponding to the amplitude and length of the auditory stimulus signal, and the values of each of the above-listed frequencies of the auditory stimulus signal.

More specifically, in this example, the set of signal features comprises a set of frequency signal features.

In order to calculate both subsets, two different averages of the EEG signal are performed: in step 204, an averaging is performed by obtaining the neural response (i.e., the "echo") of each of the 50 speech components alternating in polarity from the obtained EEG signal. Subsequently, in time, each speech component with a positive polarity is added with the following adjacent speech component with a negative polarity, and the result is divided by two. This is performed in time over the 50 speech components found within the obtained EEG signal (forming 25 additions divided by two), and the results of each addition divided by two are added altogether, forming a resulting averaged "addition" EEG signal. By performing this "addition" averaging, the power of the obtained EEG signal at the frequencies of the formants of the auditory stimulus is decreased, leaving an averaged "addition" EEG signal with an enhanced power of the obtained EEG (pre-averaging) at the fundamental frequency of the auditory stimulus.

Once the averaged "addition" EEG signal is obtained, in step 206, the following frequency signal features are calculated from the averaged "addition" EEG signal:
Spectral amplitude at the fundamental frequency F0 of the auditory stimulus, which is calculated as the averaged value of the amplitude of the FFT of the averaged "addition" EEG signal at F0 and in a frequency range of 10Hz;
Signal-to-noise ratio (SNR) at the fundamental frequency F0 of the auditory stimulus, which is calculated as the the ratio between the above-calculated spectral amplitude at F0 and the noise around it in a frequency range of 10Hz.

In parallel, in step 205, an averaging is performed by obtaining the neural response (i.e., the "echo") of each of the 50 speech components alternating in polarity from the obtained EEG signal (as in step 204). Subsequently, in time, each speech component with a positive polarity is subtracted from the following adjacent speech component with a negative polarity, and the result is divided by two. This is performed in time over the 50 speech components found within the obtained EEG signal (forming 25 subtractions divided by two), and the results of each addition divided by two are added altogether, forming a resulting averaged "subtraction" EEG signal. By performing this "subtraction" averaging, the power of the obtained EEG signal at the fundamental frequency is decreased, leaving an averaged "subtraction" EEG signal with an enhanced power of the obtained EEG signal (pre-averaging) the frequencies of the formants of the auditory stimulus.

Once the averaged "subtraction" EEG signal is obtained, in step 207, the following frequency signal features and temporal signal features are calculated from the averaged "subtraction" EEG signal:
Spectral amplitude at the formant frequencies F1 and F2 of the auditory stimulus, which is calculated as the averaged value of the amplitude of the FFT of the averaged 'subtraction' EEG signal at F1 during the first portion 32 (corresponding to the |o| of the speech component 31), and at F2 during the second portion 33 (corresponding to the |a| of the speech component 31) and in a frequency range of 10Hz;
Signal-to-noise ratio (SNR) at the formant frequencies F1 and F2 of the auditory stimulus, which is calculated as the ratio between the above-calculated spectral amplitude at F1 and F2 and the corresponding noise around each of them in a frequency range of 10Hz.

Thus, in step 208, the set of signal features comprises:
- Spectral amplitude at the fundamental frequency F₀ and the Signal-to-noise ratio (SNR) at the fundamental frequency F₀ (calculated from the averaged "addition" EEG signal); and
- Spectral amplitude at the formant frequencies F₁ and F₂ and the Signal-to-noise ratio (SNR) at the formant frequencies F₁ and F₂ (calculated from the averaged "subtraction" EEG signal);

Subsequently, in step 208, the risk that an infant presents a neural speech encoding disorder is predicted using the machine learning algorithm 14, wherein the set of signal features is input to the machine learning algorithm. In this example, as in the example of figure 1, the machine learning algorithm is a Naive Bayes classifier (NB) 14. Furthermore, in this example, the input dataset used to predict the risk that an infant presents a neural speech encoding disorder comprises the previously calculated set of signal features and metadata corresponding to the neonatal infant 20, the metadata comprising:
- AG: data corresponding to the gestational age in weeks;
- Gender: data corresponding to the sex of the baby (1 if female and 0 if male);
- Weight: data corresponding to the weight of the infant 20 in grams.
- Pc: data corresponding to the percentile of weight of the baby, according to normative tables.

In step 209, the result of the prediction performed in step 209 is obtained as the output of the Naive Bayes classifier (NB) 14, in the form of a risk parameter. In this example, the risk parameter has two possible values, "0" being the value of no risk that an infant presents a neural speech encoding disorder and "1" being the value for risk that an infant presents a neural speech encoding disorder.

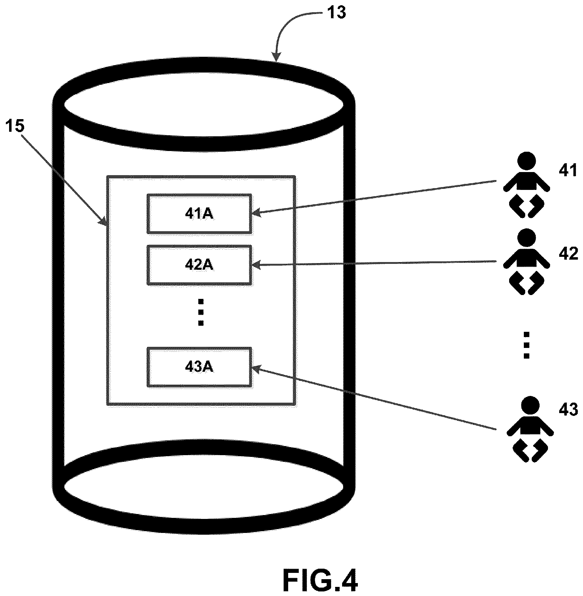

Figure 4 depicts a detail of the database 13 of the example of figure 1, wherein the database 13 comprises a data block 14 comprising the Naive Bayes classifier (NB) 14, and a data block 15 comprising a group of sets of signal features. In particular, the group of sets of signal features comprises 100 sets, of which a first set 41A, a second set 42A, and a third set 42B are depicted. Each set of signal features 41A, 42A, 43A comprises, in this example, the same set of signal features calculated in the method of figure 2 (i.e., Spectral amplitude (F0) and SNR (F0) both calculated from an averaged "addition" EEG signal, and Spectral amplitude (F1 and F2) and SNR (F1 and F2) calculated from an averaged "subtraction" EEG signal). Each set of signal features 41A, 42A, 43A have been previously obtained from an EEG signal from a different neonatal infant 41, 42, 43, all the infants aged 0-6 months old.

The data block 15 is thus used as part of a training dataset for the Naive Bayes classifier (NB) 14.

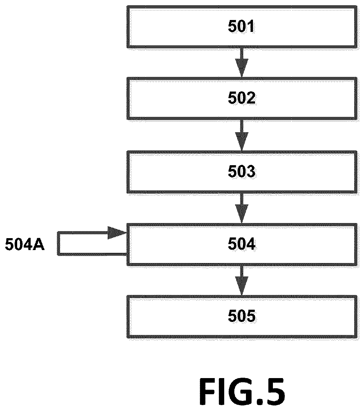

Figure 5 depicts an example of the training process of a machine learning algorithm according to the present disclosure. More specifically, in this example, the machine learning algorithm is the Naive Bayes classifier (NB) 14 of the example of figure 1.

In step 501 the computing system 13 retrieves the data block 15 comprising a group of sets of signal features from the database 13, each set corresponding to each infant of a group of infants. Each set of signal features of the retrieved group of sets of signal features has been previously obtained by, for example, a doctor by delivering an auditory stimulus to a neonatal infant of between 0 and 6 months old, and the set of signals have been calculated based on an obtained EEG signal of the infant in response to the delivery of the auditory stimulus. A further data block 16 related to data block 15 is also retrieved, the data block 16 comprising metadata related to each infant from whom each set of signal features has been obtained.

In step 502 the computing system 13 retrieves the data block 17 comprising data corresponding to a Language scale of the Bayley test, the data corresponding to each of the infants from the group of infants from which each set of signal features in data block 15 and the metadata in data block 16 has been previously obtained. That is, in this example, for each set of signal features in data block 15, data block 17 comprises a Language scale of the Bayley test, wherein the Bayley test has been performed when the corresponding infant is aged 21 months or older.

In step 503 the computing system 13 calculates, for each Language scale of the Bayley test found within the retrieved data block 17, a risk parameter corresponding to the risk that an infant presents a neural speech encoding disorder. In this example, a value of "1" is assigned to the risk parameter when there is a risk of a neural speech encoding disorder, and a value of "0" is assigned to the risk parameter if there is no risk of neural speech encoding disorder. Furthermore, in this example, for values of equal or above 89 of the Language scale of the Bayley test, the risk parameter is assigned a "0" value (i.e., no risk), and for values of below 89 of the Language scale of the Bayley test, the risk parameter is assigned a "1" value (i.e., risk).

In step 504 the computing system 13 trains iteratively 504A in a supervised manner the Naïve Bayes classifier (NB) 14 found in database 13 by inputting the retrieved data block 15 and data block 16 as an input dataset to the Naive Bayes classifier (NB) 14. The classifier 14 is iteratively trained 504A using each set of signal features and metadata corresponding to an infant in the data blocks 15 and 16, and using an output dataset comprising the corresponding risk parameter previously calculated for each of the corresponding infants.

In step 505, the Naive Bayes classifier (NB) 14 found in database 13 is trained and can be used to predict a risk that an infant presents a neural speech encoding disorder using a new set of signal features as an input for the classifier 14.

For reasons of completeness, various aspects of the present disclosure are set out in the following numbered clauses:
Clause 1. A computer implemented method of predicting a risk that an infant presents a neural speech encoding disorder, the method comprising the steps of:
   - obtaining an electroencephalography, EEG, signal of an infant aged 0 to 6 months old in response to an auditory stimulus, wherein the auditory stimulus comprises a speech component representing at least two phonemes, wherein at least one phoneme is the 'a' phoneme;
   - obtaining a set of signal features from the EEG signal, the set of signal features relating to the neural speech encoding performed by the brain of the infant;
   - predicting a risk that an infant presents a neural speech encoding disorder using a machine learning algorithm, wherein the set of signal features is input to the machine learning algorithm;
   wherein the machine learning algorithm is trained using a supervised training and a training data set, the training data set comprising:
   - an input dataset comprising a set of signal features obtained from an EEG signal of each infant of a group of infants aged 0 to 6 months old; and
   - an output dataset comprising a risk parameter for each infant of the group of infants, the risk parameter having at least two possible values, wherein a first value represents a high risk of a neural speech encoding disorder and a second value represents a low risk of a neural speech encoding disorder;
   - wherein the value of the risk parameter is based on data corresponding to a Language scale of the Bayley test, the Language scale of the Bayley test being obtained from each one of the corresponding infants from which the set of signal features in the input dataset are obtained, and wherein the infants are aged 21 months old or older at the time of the obtaining of the Language scale of the Bayley test.
Clause 2. The computer-implemented method according to clause 1, further comprising:
   - obtaining at least two portions of the obtained EEG signal, the at least two portions having an alternate polarity in time between them;
   - obtaining an averaged EEG signal by averaging the obtained at least two portions;
   and wherein the set of signal features is obtained from the averaged EEG signal.
Clause 3. The computer-implemented method according to clause 2, wherein a plurality of portions of the obtained EEG signal is obtained, a first set of the plurality of portions having an alternate polarity with respect of a second set of the plurality of portions, and wherein the average EEG signal is obtained by averaging the plurality of portions.
Clause 4. The computer-implemented method according to any one of clauses 2 or 3, wherein the averaging is performed by subtracting each obtained portion of the first set from another obtained portion of the second set, and dividing the result of each subtraction by two.
Clause 5. The computer-implemented method according to any one of clauses 2 or 3, wherein the averaging is performed by adding each obtained portion of the first set from another obtained portion of the second set, and dividing the result of each subtraction by two.
Clause 6. The computer implemented method according to any one of clauses 1 to 5, wherein at least one signal feature from the obtained set of signal features is a temporal signal feature.
Clause 7. The computer implemented method according to clause 6, wherein the temporal signal feature is at least one of: RMS, stimulus-response cross-correlation, neural lag, or pitch strength.
Clause 8. The computer implemented method according to any one of clauses 1 to 7, wherein at least one signal feature from the obtained set of signal features is a frequency signal feature.
Clause 9. The computer implemented method according to clause 8, wherein the frequency signal feature is at least one of: a spectral amplitude of the obtained or averaged EEG signal at the fundamental frequency of the auditory stimulus; or a signal-to-noise ratio parameter.
Clause 10. The computer implemented method according to any of clauses 1 to 9, wherein the machine learning algorithm is a Linear Model type algorithm.
Clause 11. The computer implemented method according to clause 10, wherein the value of the risk parameter is based on data corresponding to the Expressive Communication subscale of the Language scale of the Bayley test.
Clause 12. The computer implemented method according to any of clauses 1 to 9, wherein the machine learning algorithm is an Instance-based type algorithm.
Clause 13. The computer implemented method according to any of clauses 1 to 9, the value of the risk parameter is based on data corresponding to the Receptive Communication subscale of the Language scale of the Bayley test, and the machine learning algorithm is a Tree-Based type algorithm.
Clause 14. The computer implemented method according to clauses 13, wherein the Tree-Based type algorithm is an Extreme Tree Classifier.
Clause 15. A data processing system comprising a processor configured to perform the steps of the method of any of clauses 1 to 14.
Clause 16. A computer program product comprising instructions which, when the program is executed by a computer, cause the computer to carry out the steps of the method of any of clauses 1 to 14.
Clause 17. The computer program product according to clause 16, embodied on a storage medium.
Clause 18. The computer program product according to clause 16, carried on a carrier signal.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow. If reference signs related to drawings are placed in parentheses in a claim, they are solely for attempting to increase the intelligibility of the claim, and shall not be construed as limiting the scope of the claim.

Further, although the examples described with reference to the drawings comprise computing apparatus/systems and processes performed in computing apparatus/systems, the invention also extends to computer programs, particularly computer programs on or in a carrier, adapted for putting the system into practice.

## Claims

1. A computer implemented method of predicting a risk that an infant presents a neural speech encoding disorder, the method comprising the steps of:
- obtaining an electroencephalography, EEG, signal of an infant aged 0 to 6 months old in response to an auditory stimulus, wherein the auditory stimulus comprises a speech component representing at least two phonemes, wherein at least one phoneme is the 'a' phoneme;
- obtaining a set of signal features from the EEG signal, the set of signal features relating to the neural speech encoding performed by the brain of the infant;
- predicting a risk that an infant presents a neural speech encoding disorder using a machine learning algorithm, wherein the set of signal features is input to the machine learning algorithm;
wherein the machine learning algorithm is trained using a supervised training and a training data set, the training data set comprising:
- an input dataset comprising a set of signal features obtained from an EEG signal of each infant of a group of infants aged 0 to 6 months old; and
- an output dataset comprising a risk parameter for each infant of the group of infants, the risk parameter having at least two possible values, wherein a first value represents a high risk of a neural speech encoding disorder and a second value represents a low risk of a neural speech encoding disorder;
- wherein the value of the risk parameter is based on data corresponding to a Language scale of the Bayley test, the Language scale of the Bayley test being obtained from each one of the corresponding infants from which the set of signal features in the input dataset are obtained, and wherein the infants are aged 21 months old or older at the time of the obtaining of the Language scale of the Bayley test.

2. The computer-implemented method according to claim 1, further comprising:
- obtaining at least two portions of the obtained EEG signal, the at least two portions having an alternate polarity in time between them;
- obtaining an averaged EEG signal by averaging the obtained at least two portions;
and wherein the set of signal features is obtained from the averaged EEG signal.

3. The computer-implemented method according to claim 2, wherein a plurality of portions of the obtained EEG signal is obtained, a first set of the plurality of portions having an alternate polarity with respect of a second set of the plurality of portions, and wherein the average EEG signal is obtained by averaging the plurality of portions.

4. The computer-implemented method according to any one of claims 2 or 3, wherein the averaging is performed by subtracting each obtained portion of the first set from another obtained portion of the second set, and dividing the result of each subtraction by two.

5. The computer-implemented method according to any one of claims 2 or 3, wherein the averaging is performed by adding each obtained portion of the first set from another obtained portion of the second set, and dividing the result of each subtraction by two.

6. The computer implemented method according to any one of claims 1 to 5, wherein at least one signal feature from the obtained set of signal features is a temporal signal feature.

7. The computer implemented method according to claim 6, wherein the temporal signal feature is at least one of: RMS, stimulus-response cross-correlation, neural lag, or pitch strength.

8. The computer implemented method according to any one of claims 1 to 7, wherein at least one signal feature from the obtained set of signal features is a frequency signal feature.

9. The computer implemented method according to claim 8, wherein the frequency signal feature is at least one of: a spectral amplitude of the obtained or averaged EEG signal at the fundamental frequency of the auditory stimulus; or a signal-to-noise ratio parameter.

10. The computer implemented method according to any of claims 1 to 9, wherein the machine learning algorithm is a Linear Model type algorithm, and the value of the risk parameter is based on data corresponding to the Expressive Communication subscale of the Language scale of the Bayley test.

11. The computer implemented method according to any of claims 1 to 9, the value of the risk parameter is based on data corresponding to the Receptive Communication subscale of the Language scale of the Bayley test, and the machine learning algorithm is a Tree-Based type algorithm.

12. A data processing system comprising a processor configured to perform the steps of the method of any of claims 1 to 11.

13. A computer program product comprising instructions which, when the program is executed by a computer, cause the computer to carry out the steps of the method of any of claims 1 to 11.

14. The computer program product according to claim 13, embodied on a storage medium.

15. The computer program product according to claim 13, carried on a carrier signal.
